# EUROPEAN PATENT SPECIFICATION

(11) **EP 2 538 924 B1**
(45) Date of publication and mention of the grant of the patent: **21.05.2014**
(21) Application number: 10717277.7
(22) Date of filing: 14.04.2010
(51) Int. Cl.: A61K 31/4422, A61K 9/20, A61K 31/403

(54) **SOLID PHARMACEUTICAL FORMULATIONS OF RAMIPRIL AND AMLODIPINE BESYLATE, AND THEIR PREPARATION**
FESTE PHARMAZEUTISCHE FORMULIERUNGEN AUS RAMIPRIL UND AMLODIPINBESYLAT SOWIE IHRE HERSTELLUNG
FORMULATIONS PHARMACEUTIQUES SOLIDES DE RAMIPRIL ET DE BÉSYLATE D'AMLODIPINE ET LEUR PRÉPARATION

(30) Priority: 24.02.2010 IN DE04172010
(43) Date of publication of application: 02.01.2013
(73) Proprietor: Sanofi-Aventis Deutschland GmbH, 65929 Frankfurt am Main (DE)
(72) Inventor: JAISWAL, Nilesh, Goa 403 722 (IN); KHULLAR, Praveen, Goa 403 722 (IN); KULKARNI, Amol, Goa 403 722 (IN); PRAJAPATI, Dilip, Goa 403 722 (IN)
(74) Representative: Taravella, Brigitte
(86) International application number: PCT/IB2010/051616
(87) International publication number: WO 2011/104588

(56) References cited:
- WO-A1-2005/120502
- WO-A1-2007/040511
- US-A1- 2008 096 863

## Description

### FIELD OF THE INVENTION

The present invention is directed to solid stable pharmaceutical compositions comprising ramipril, amlodipine besilate and pharmaceutically acceptable excipients, to their preparation and to their therapeutic application. This means that in this composition, the two active ingredients are together in single dosage form.

### BACKGROUND OF THE INVENTION.

Amlodipine is a calcium channel blocker developed for the treatment of hypertension and other medical indications as disclosed in USP 4,572,909 and USP 4,879,303. Its chemical name is 3-ethyl-5-methyl-(+-)-2-[(2-aminoethoxy) methyl]-4-(2- chlorophenyl)-1,4-dihydro-6-methylpyridine-3,5-dicarboxylate.

Amlodipine is marketed as the monobenzenesulfonate salt, amlodipine besylate under the trade name Norvasc® or Istin ®. It is available as oral tablets in strengths of 2.5 mg, 5 mg and 10 mg. The inactive ingredients in the Norvasc® tablets include microcrystalline cellulose, dibasic calcium phosphate anhydrous, sodium starch glycolate and magnesium stearate.

Amlodipine besylate is slightly soluble in water and has an absolute bioavailability of 64-90%.

Ramipril, an angiotensin converting enzyme (ACE) inhibitor, and its physiologically acceptable salts are disclosed in U.S. Pat. No.5,061,722. Chemically, it is designated as (2S,3aS,6aS)-1{-(S)-N-([(S)-1-carboxy-3-phenylpropyl]alanyl} octahydro-cyclopenta pyrrole-2-carboxylic acid 1-ethyl ester and is used for the treatment of hypertension, heart failure, and nephropathy. Ramipril is susceptible to degradation by hydrolysis to ramipril diacid[(2S,3aS,6aS)-1-[(S)-2-[[(S)-1-carboxy-3-phenylpropyl]amino]propanoyl] octahydrocyclopenta [b]pyrrole-2-carboxylic acid ("Impurity E"). This is an active metabolite of ramipril and therefore may not necessarily require control in the formulation. It is also susceptible to cyclization to ramipril diketopiperazine [ethyl (2S)-2-[(3S,5aS,8aS,9aS)-3-methyl-1,4-dioxodecahydro-2H-cyclopenta[4,5]pyrrolo[1,2-a]pyrazin-2-yl]-4-phenylbutanoate ("Impurity D"). These impurities are defined in European Pharmacopoeia.

It is always desirable to combine multiple active ingredients in a single pharmaceutical composition. Inclusion of multiple ingredients in a single composition generally reduces costs and provides the convenience of consuming a single medication rather than multiple medications for treating individual symptoms. The inherent advantage of fixed-dose combination therapy resides in its improving compliance because fewer pills are required. The therapeutic treatment becomes simplified and there are more potential for improved outcomes.

However, a combination of active ingredients is not without drawbacks and challenges to be faced in formulation development.

Certain physical properties of the drugs and specifically photo and chemical stability, imparts a great challenge in developing formulations suitable for preparing a tablet having reduced levels of total impurities on long term stability.

Amlodipine is, for example, with relatively low bulk densities. The active exhibits poor flow and low aqueous solubility, can further contribute to problems such as tableting or uniformity of dosage units. It is also a sticky and highly cohesive material. Amlodipine besilate is weakly basic in nature and prone to degradation in moist conditions and under exposure to light. The major degradant formed under light exposure is Impurity-D which is a photo-degradant and is formed in oxidative conditions. The formation of photo-degradant is more, when the pH of the microenvironment decreases.

Ramipril has certain undesirable flow characteristics, for example, is sticky and can adhere to surfaces such as tablet punch faces and dies, causing problems in tableting, especially on a high speed tablet press. Ramipril is weakly acidic in nature and is very sensitive to degradation under alkaline conditions, oxidative and wherein moisture is present. The degradation of ramipril is believed to occur mainly via two pathways: (a) hydrolysis to ramipril-diacid; and (b) cyclization or condensation to ramipril-diketopiperazine (ramipril-DKP), which is profoundly formed more when the pH of the microenvironment increases. In addition, the stability of a composition might be compromised due to incompatibility of an active with an essential excipient or even between a second active itself.

In addition, the stability of a composition might be affected due to incompatibility of two active(s) with one or more essential excipients.

In US 2008/0096863, it has been found that a stable composition of a combination of amlodipine and an ACE inhibitor such as Ramipril can be prepared using basifying agents without the need of physical separation of the two active ingredients and without compromising the stability.

Thus, there is a need for new stable solid dosage form comprising the specific combination of Ramipril and Amlodipine besilate.

There is also a need to formulate a simplified stabilized fixed dose combination of Ramipril and Amlodipine which can have better industrial scale manufacturing feasibility and on the same hand also exhibit better stability than the individual individual product specifically with respect to Ramipril Diketopiperazine (DKP) and impurity D of Amlodipine.

In addition to stability, when formulating solid fixed dose combination, the objective is to provide a patient-convenient combination dosage form of active ingredients that is bioequivalent to the corresponding free-combination of the same active ingredients.

As used herein, "fixed-dose-combination or FDC" refers to a combination of two drugs or active ingredients presented in a single dosage unit such as a tablet or oral dosage form.

Further as used herein, "free- combination" refers to a combination of two drugs or active ingredients dosed simultaneously but as two dosage units.

As a result of these complex biopharmaceutical properties, development of a fixed-combination dosage form of Ramipril and Amlodipine besilate that is bioequivalent to a free-combination thereof is challenging.

Accordingly, a fixed-combination solid dosage formulation of Ramipril and Amlodipine besilate that is stable and bioequivalent to the corresponding free-combination would be desirable.

Pharmaceutical compositions of such a combination of Amlodipine and Ramipril are difficult to prepare due to the incompatibility of the active ingredients.

Ramipril may show more stability related problems during formulation than that of Amlodipine. Ramipril shows a tendency to be unstable in pharmaceutical formulations, pH of the formulation depending on the excipients used, the manufacturing process and storage; high temperature, humidity and compression are the factors that determine the stability of the formulations with Ramipril.

Another challenge faced is homogeneity of Amlodipine in a lubricated blend as the content of Amlodipine in the total tablet weight should be very low compared to the high amount of Ramipril.

The object of the present invention is to overcome at least partly the above mentioned drawbacks which are basically 1) To manufacture a physically non-separable simplified formulation 2) to provide an improved formulation specifically without any basifying agent to achieve the stability of the product.

### SUMMARY OF THE INVENTION.

The object of then invention is related to a stable solid oral pharmaceutical fixed dose composition in form of a tablet comprising Ramipril, amlodipine besilate and pharmaceutically acceptable excipients, wherein ramipril under the form of coated granules is embedded in an extragranular matrix comprising amlodipine besilate, wherein the pharmaceutically acceptable excipients are selected from the group consisting of diluent, disintegrant, antiadherent, binder lubricant and mixture thereof and wherein the pH of the excipient mixture, after dispersion in 50 ml of purified water, is from 6,1 to 6,3 preferably 6,2.

In a preferred embodiment, the solid composition takes the form of a monolayer tablet with a pH of 4,7 to 5,0 , preferably 4,9, preferably, the tablet is further package in duplex blister pack.

In a preferred embodiment of the composition, Ramipril represents between about 2,0% and about 20% by weight of the total composition, preferably about 2,5% or about 10% by weight of the total composition.

In a preferred embodiment, amlodipine besilate represents between about 5% and about 10% by weight of the total composition and preferably about 7.0%.

The pharmaceutically acceptable excipients are selected from the group consisting of diluent, disintegrant, antiadherent, binder lubricant and mixture thereof and wherein the pH of the excipient mixture is from 6,1 to 6,3 preferably 6,2.

In a preferred embodiment the amount of ramipril is comprised between 2,5mg and 10mg of the total weight of the tablet, preferably 2,5mg or 10mg.

In a preferred embodiment the amount of amlodipine besilate is comprised between 5mg and 10mg of the total weight of the tablet, preferably 7mg.

In a preferred embodiment the solid composition is under the form of a tablet wherein the total weight of the tablet is between 80mg and 100mg, preferably 100mg.

In a preferred embodiment the composition is having less than about 3.19% (w/w) of Ramipril DKP and Amlodipine Impurity-D of less than about 0.14% (w/w) after 6 months at 40°C & 75% RH.

Another object of the invention is a process for the preparation of a stable oral pharmaceutical composition comprising Ramipril and amlodipine besilate, wherein the process comprises the steps of:
1) granulating Ramipril and one or more pharmaceutically acceptable excipients, with aqueous solution containing a binder, to form granules,
2) drying the granules;
3) separately blending amlodipine besilate with pharmaceutically acceptable excipients,
4) mixing the Ramipril granules of step ii with the amlodipine besilate blend of step iii);
5) lubricating the blend of step iv); optionally after a pre-lubricating step; and
6) compressing the mixture into tablets
wherein the group of pharmaceutically acceptable excipients used in step 1 and 3) of the process are selected from the group consisting of diluent, disintegrant, antiadherent, binder lubricant and mixture thereof and wherein the pH of the excipient mixture is from 6,1 to 6,3 preferably 6,2

In a preferred embodiment the pharmaceutically acceptable excipients are selected from hydroxyl propyl methyl cellulose, pregelatinised starch, microcrystalline cellulose and sodium stearyl fumarate

In a preferred embodiment the process further comprises the step of coating the tablet and packaging in suitable duplex blister pack.

In a preferred embodiment the process of the invention comprises the step of
1) add Ramipril as Hydroxy propyl methyl cellulose granules
   1a) Co-sift Ramipril granules with half portion of pregelatinised starch.
2) Co-sift Amlodipine Besilate with half portion of microcrystalline cellulose
3) Mix step 3 material with step 2 materials & blend for 20 min in blender to achieve homogeneity.
   3a) Co-sift remaining portions of microcrystalline cellulose & pregelatinised starch through # 40 mesh.
   3b) Blended step 4 and 5 for 15 minutes at 18 RPM in a suitable blender.
4) Lubricated the blend of step 6 using sodium stearyl fumarate.
5) Compress the blend into tablets using suitable punches on a tablet press.

Another object of the invention is the use of a blend of pharmaceutically acceptable excipients selected from the group consisting diluent, disintegrant, antiadherent, binder lubricant and mixture thereof, the blend having a pH being from 6,1 to 6,3 preferably 6,2, for manufacturing a stable solid oral pharmaceutical fixed dose composition comprising Ramipril and amlodipine besilate, wherein the solid composition takes the form of a monolayer tablet with a pH of 4,7 to 5,0 , preferably 4,9.

In a preferred embodiment the pharmaceutically acceptable excipients are selected from hydroxyl propyl methyl cellulose, pregelatinised starch, microcrystalline cellulose and sodium stearyl fumarate.

In a preferred embodiment ramipril is under the form of coated granules embedded in an extragranular matrix comprising amlodipine besilate.

Another object of the invention is the use of Ramipril and Amlodipine besilate in the manufacture of a medicament for the treatment of arterial hypertension and prevention of other cardiovascular diseases such as myocardial infarction, cerebrovascular disorders and cardiac and renal insufficiency wherein said medicament is in the stable solid fixed dose composition according to anyone of claim 1 to 10.

### DETAILED DESCRIPTION OF THE INVENTION.

The stable solid oral pharmaceutical fixed dose composition of the invention comprises Ramipril, Amlodipine besilate and pharmaceutically acceptable excipients, wherein Ramipril is physically separated from amlodipine besilate and is developed without addition of any basifying agents.

In a specific embodiment the pH of the combination is between 4.7-5.0 or preferably about 4.9.

This fixed dose combination solid dosage form is particularly advantageous since amlodipine besilate does not undergo degradation and this combination product shows reduced and controlled impurities even lesser than with regards to individual reference products of same dose when subjected in finished pack.

The preparation of stable pharmaceutical formulations of ramipril is complicated since it is susceptible to certain types of pH dependant degradation.

Formation of Ramipril DKP is profoundly dependant on pH of the formulation, especially at very low pH values. Reported literatures say that increasing the pH of microenvironment of Ramipril minimizes the formation of DKP impurity.

Whereas with Amlodipine, the photodegradant Impurity-D of Amlodipine is formed more in alkaline environment and less in weak acidic environment. The pH of the Istin tablets is 7.2 whereas the pH of Cardace is ∼4.2-4.3. (Against pH 4.7-5.0 in Ramipril+Amlodipine tablets).

The inclusion of extragranular Amlodipine besylate (specifically-dry mixed) being basic in nature in the FDC product imparted the pH of the FDC tablet between 4.7-5.0. Thus, the stability of the Ramipril in the combination formulation was improved.

On the same hand due to acidic nature of ramipril the stability of amlodipine was improved with respect to formation of Impurity-D (a photodegradant which is formed more in alkaline environment and less in acidic environment).

Therefore in this way, synergistic pH-stabilizing effect of the two actives, and appropriate selection of direct compressible grade (low moisture grade) of excipients enabled the formulation of a degradation sensitive combination product .The out come was that the (Impurity D of Amlodipine and Ramipril DKP) were found to be less than their individual innovator's product.

### Preferred Ramipril and Amlodipine besilate compositions.

The two actives are present in a single unit dosage form, such as tablets or pellets, wherein Ramipril is physically separated from amlodipine besilate.

There was a need to formulate the two actives in tablet dosage form due to proven and well known said advantages over capsule dosage form which are already marketed under brand name of RAMISTAR-A capsules, NAPRIX-A capsules and STAMACE capsules by Lupin Laboratories India, Libb's Laboratories Brazil and Dr. Reddy's Laboratories India respectively. Tablet dosage forms do not require specialized manufacturing conditions like low humidity during processing owing to presence of gelatine in the capsule shells.

Pharmaceutically acceptable excipients are selected from the group consisting of diluent, disintegrant, antiadherent, binder lubricant and mixture thereof and wherein the pH of the excipient mixture is from 6,1 to 6,3 preferably 6,2.

Pharmaceutically acceptable additives suitable for use in the present invention with amlodipine besylate & ramipril are selected from suitable diluents such as microcrystalline cellulose, pregelatinized starch and suitable lubricants such as sodium stearyl fumarate or sodium stearyl fumarate.

In a preferred form of the tablet, ramipril is present under the form of granules embedded in an extragranular matrix comprising amlodipine besilate added extra granularly along with excipients.

Ramipril is granulated with a binder such as HPMC and granules dried and blended with excipients along-with amlodipine added extra granularly.

Preferred compositions of the present invention contain one or more of the following components in the indicated concentration range (% by weight): Ramipril (2.5-10%), amlodipine besylate (7%), diluent about 35%-50% disintegrant about 40-45% 44%, binder about 0,4-2% 0.44 -1.77% and lubricant about 0,2-0,6%

More preferred compositions of the present invention contain one or more of the following components in the indicated concentration range (% by weight): Ramipril (2.5-10%), amlodipine besylate (7%), diluent about 36.73%-45.56%, disintegrant about 44%, binder about 0.44 -1.77% and lubricant about 0.5%.

The final pH observed for the Ramipril and Amlodipine fixed dose combination tablets formula of the invention is from 4.7 to 5.0 preferably about 4.9, whereas pH of individual reference product was found to be; 7.2 for amlodipine besilate (Istin tablets) ∼4.2-4.3 for Ramipril (Cardace).

The final primary packaging material used is blister packaging i.e. duplex (300micPVC/90gsmPVdC). These materials will avoid increasing generation of Impurity-D in amlodipine thereby inhibiting direct exposure of sun light on drug product.

### Stability performances.

The main impurity to be controlled in the composition of the invention is Impurity D known in the art as 3-ethyl-5-methyl-2-[(2-aminoethoxy)methyl]-4-(2-chlorophenyl)-6- methylpyridine-3,5-dicarboxylate.

Ramipril and Amlodipine Besylate are well known in prior art for chemical instability therefore prone to degradation. Based on extensive drug excipient compatibility studies and excipients characterization the two unstable actives were formulated together into a stable dosage form, wherein it was observed that the test formulation was found to be better than the individual reference products with respect to impurities

In the compositions according to the invention, 3.19 % (w/w) of Ramipril DKP impurity (potential degradant) was found after 6 months at 40°C/75%RH, with respect to individual reference product Cardace which was 3.29% when Ramipril and amlodipine are physically separated. When impurity profiles of the test product was compared with marketed brands like Ramistar-A and or Stamace, then it was observed that the Ramipril DKP impurity and Amlodipine-D impurity was significantly less in the test formulation.

The oral pharmaceutical compositions of the present invention were subjected to accelerated stability studies at the following conditions; 40°C & 75 % relative humidity RH and 25°C & 60 % RH. These were evaluated on the basis of assay, in vitro dissolution, moisture content and related substances measured between initial and 6-months time points for Ramipril as well as amlodipine besylate.

The specific compositions of the invention have a lowering impact on the degradation profile of amlodipine besylate.

The results provide a significant decrease in the Amlodipine Imp D & Ramipril DKP when Ramipril and Amlodipine both are physically separated.

Specifically, it was investigated that impurity levels in the Amlodipine is quite low and within the target limit in the formula where Ramipril granulated and Amlodipine was added in the extragranular portion. This composition shows impurity levels much lower than that of reference products. Addition of Amlodipine extragranularly imparted significant stability to the formulation because this leads to minimize the particle to particle contact between the two actives, which are other wise individually highly unstable if formulated with appropriate excipients.

Formulating the two individual active ingredients in one singe dosage form, was a challenge for a chemical instability point of view.

This was achieved by proper selection of excipients or placebo blend in appropriate proportions and mixed with the active ingredients. It was the pH of the placebo which played an important role in creating a desired pH at the microenvironment around the actives which stabilized the product.

Therefore different placebo blend of different pH was used in the formulation and put on stability in order to study the effect of "pH of placebo blend" versus percentage of potential degradants in the fixed dose combination product.

The stability results clearly indicate significant contribution of pH of placebo on the stability of the formulation. The choice of appropriate pH of placebo in the formulation now depends upon the level of impurities in the product (i.e. Impurity D originating from Amlodipine and Ramipril DKP from Ramipril).

Use of placebo imparting pH between 6,1 and 6,3 preferably 6.2 was chosen finally since the level of Ramipril DKP was least in comparison the formulations having pH of Placebo of 6.4 & 6.7.

There was significant contribution of pH of microenvironment around actives which was responsible for the stabilization of the combination product and this inference could not be anticipated.

The unit weight of tablets placebo were added to 50 ml of purified water and stirred manually using clean glass rod and pH noted using suitable calibrated pH-meter.

### Homogeneity and uniformity performances.

After stabilization of the product, development of a robust and scalable formulation was a challenge.

Indeed, the purpose was then to achieve homogeneity and uniformity, i.e. to match the bulk densities of the Ramipril granules and extra granular portion of the formulation in-order to achieve proper homogeneity of active (especially Amlodipine).

Since the bulk density of Amlodipine and Microcrystalline cellulose was found to be 0.313 g/ml and 0.347 g/ml respectively so it was difficult to match with Ramipril granules whose density was found to be 0.277 g/ml.

Therefore there was a need of increasing the bulk density of Ramipril granules.

This was done by mixing with much bulkier excipient (herewith as pregelatinised starch) so that the bulk density of Ramipril granules and pregelatinised starch reaches near the bulk density of extra granular portion (herewith Amlodipine besilate+ microcrystalline cellulose).

Then to achieve proper homogeneity and improve the distribution of actives, the co-sifting mixing methodologies based on bulk densities was chosen. The results with this method were better than with the "geometrically" mixing method.

### Dissolution performances.

The "dissolution performance" of a tablet containing the combination oframipril and amlodipine besilate is compared to the dissolution performance of each reference product in multimedia dissolution conditions such as 0.1 N HCl, pH 4.5 acetate buffer and pH 6.8 phosphate buffer. The progress of dissolution is monitored at various time points between initial point and 60 minutes.

The results show that the dissolution performances of the tablet containing the combination of and amlodipine besilate of the invention are equivalent to the dissolution performances of the tablets containing each active ingredient ramipril or amlodipine alone.

### Bioequivalence.

The bioequivalence study was achieved between Test Product: Fixed Dose Combination of Ramipril 10 mg and Amlodipine Besylate 5 mg Tablets of the invention, and free dose combination of Reference Product: Cardace® (Ramipril) Tablets 10 mg of Aventis Pharma Ltd., India and ISTIN® (Amlodipine Besylate) Tablets 5 mg of Pfizer, UK, under fasting condition in normal, healthy, adult, male human subjects, in a randomized crossover study.

### Methodology:

A total of 46 adult, healthy male subjects were enrolled in the study. The subjects were confined in the facility at least 10 hours before dosing until 48 hours post-dose during each period. After an overnight fast of at least 10 hours, subjects were administered the study drug (a single oral dose of the test product or both the reference products as per randomization schedule) with 240 mL of water in each period. A total of 29 blood samples (6 mL each) were collected from the subjects during each study period from pre-dose to 216.00 hours (post-dose). Samples at 72.00, 120.00, 168.00 and 216.00 hours post-dose were collected on ambulatory basis. Analysis of plasma concentrations of Ramipril, Ramiprilat and Amlodipine was done by a validated LC-MS/MS analytical method. A non compartmental method was used to calculate the pharmacokinetic parameters using drug concentration time profile. Statistical comparison of the pharmacokinetic parameters of the formulations was performed to assess the bioequivalence of Ramipril, Ramiprilat and Amlodipine.

### Conclusion

Based on statistical analysis of Ramipril, Ramiprilat and Amlodipine it is concluded that the Test Product: RAMIPRIL & AMLODIPINE TABLETS 10/5 mg is bioequivalent with the Reference Product (1): Cardace (Ramipril Tablets I.P) and the Reference Product (2): ISTIN [Amlodipine (as besilate)] in terms of rate and extent of absorption under fasting condition.

### Method of manufacture.

The process for the preparation of a stable oral pharmaceutical composition comprising ramipril and amlodipine besilate, comprises the following steps:
1. granulating Ramipril and one or more pharmaceutically acceptable excipients, with aqueous solution containing a binder, to form granules and drying the granules,
2. separately blending amlodipine besilate with pharmaceutically acceptable excipients,
3. mixing the Ramipril granules of step 2 with the amlodipine besilate blend of step 3);
4. lubricating the blend of step 3); optionally after a pre-lubricating step; and
5. Compressing the mixture into tablets.

Preferably in step 1) acceptable excipients are selected from the group consisting of microcrystalline cellulose, croscarmelose sodium. Preferably acceptable binder, are selected from the group consisting of cellulose derivatives such as hydroxypropyl methylcellulose.

In a preferred embodiment to achieve homogeneity of the tablets, the process comprises the following steps
1) add Ramipril as Hydroxy propyl methyl cellulose granules 1a) Co-sift Ramipril granules with half portion of pregelatinised starch.
2) Co-sift Amlodipine Besilate with half portion of microcrystalline cellulose
3) Mix step 3 material with step 2 materials & blend for 20 min in blender to achieve homogeneity.
3a) Co-sift remaining portions of microcrystalline cellulose & pregelatinised starch through # 40 mesh.
3b) Blended step 4 and 5 for 15 minutes at 18 RPM in a suitable blender.
4) Lubricated the blend of step 6 using sodium stearyl fumarate.
5) Compress the blend into tablets using suitable punches on a tablet press.

### Examples.

### Example 1.Composition using placebo of pH 6.2

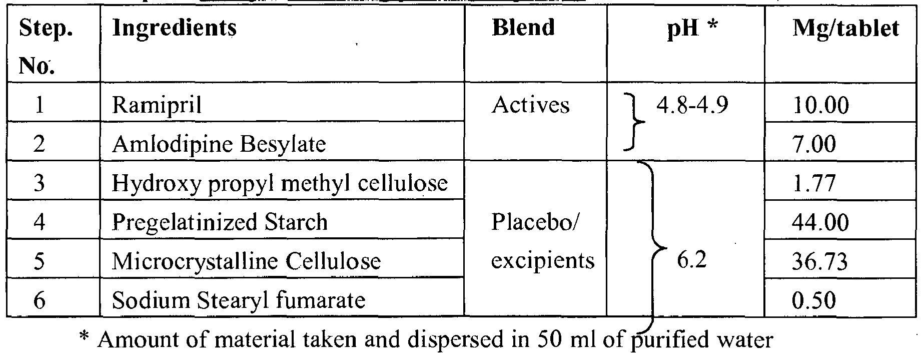

### PROCEDURE:

1. Ramipril added as Hydroxy propyl methyl cellulose granules
2. Geometrically mix Ramipril granules with pregelatinised starch.
3. Geometrically mix Amlodipine Besilate & microcrystalline cellulose & mix them with step 2 materials & blend for 20 min in blender to achieve homogeneity.
4. Lubricate the blend using sodium stearyl fumarate.
5. Compress the blend into tablets using suitable punches on a tablet press.
6. Tablets were packed in opaque white duplex and subjected for 6 month accelerated stablity studies which were analyzed at initial and 6 month intervals for percentage of potential degradants

**Table No.1**

| Period/Stability Condition | Ramipril+Amlodipine tablets | |
|---|---|---|
| | Amlodipine-D impurity | Ramipril DKP Impurity |
| Initial | Not detected | 0.12% |
| 6 M at 40°C & 75%RH | 0.1 % | 3.19% |

The study of the effect of pH of placebo on the stabilization of the two actives in the formulation further trials was tested by using different proportion of microcrystalline cellulose and pregelatinised starch.

### Example 2 (comparative): Composition using placebo of pH 6.40

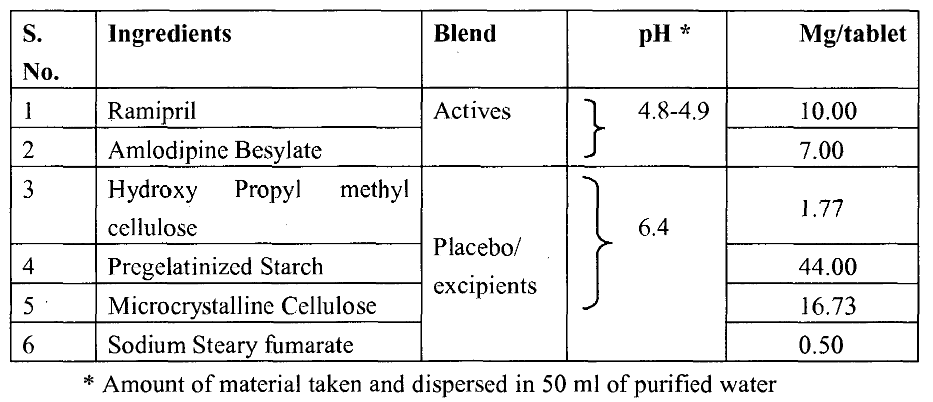

### PROCEDURE:

1. Ramipril added as Hydroxy propyl methyl cellulose granules
2. Geometrically mix Ramipril granules with microcrystalline cellulose
3. Geometrically mix Amlodipine Besilate & pregelatinised starch & mix them with step 2 materials & blend for 25 min in blender to achieve homogeneity.
4. Lubricate the blend using sodium stearyl fumarate.
5. Compress the blend into tablets using suitable punches on a tablet press.
6. Tablets were packed in opaque white duplex and subjected for 6 month accelerated stablity studies which were analyzed at initial and 6 month intervals for percentage of potential degradants

**Table No.2**

| Period/Stability Condition | Ramipril+Amlodipine tablets | |
|---|---|---|
| | Amlodipine-D impurity | Ramipril DKP Impurity |
| Initial | Not detected | 0.17% |
| 6 M at 40°C & 75%RH | 0.13 % | 9.8% |

### Example. 3 (comparative): Composition using placebo of pH 6.7

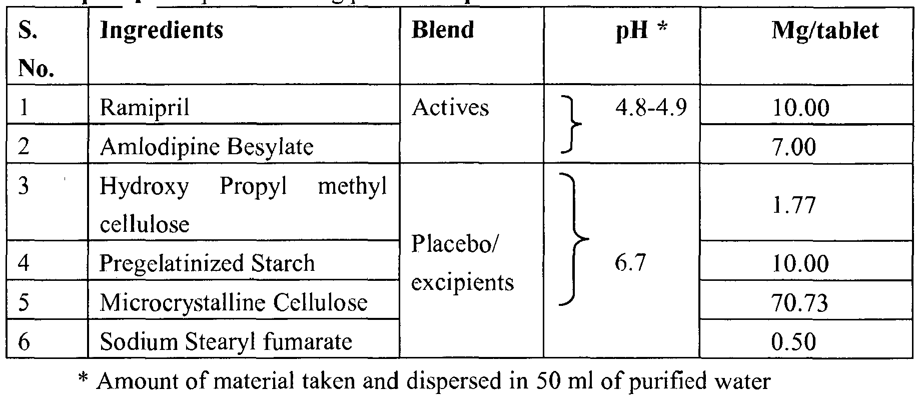

### PROCEDURE:

1. Ramipril added as Hydroxy propyl methyl cellulose granules
2. Geometrically mix Ramipril granules with pregelatinised starch.
3. Geometrically mix Amlodipine Besilate & microcrystalline cellulose & mix them with step 2 materials & blend for 25 min in blender to achieve homogeneity.
4. Lubricate the blend using sodium stearyl fumarate.
5. Compress the blend into tablets using suitable punches on a tablet press.
6. Tablets were packed in opaque white duplex and subjected for 6 month accelerated stablity studies which were analyzed at initial and 6 month intervals for percentage of potential degradants

**Table No.3**

| Period/Stability Condition | Ramipril+Amlodipine tablets | |
|---|---|---|
| | Amlodipine-D impurity | Ramipril DKP Impurity |
| Initial | Not detected | Not detected |
| 6 M at 40°C & 75%RH | 0.1 % | 3.46% |

The stability results of example no. 1, 2 and 3 clearly indicate significant contribution of pH of placebo on the stability of the formulation. The choice of appropriate pH of placebo in the formulation now depends upon the level of impurities in the product (i.e. Impurity D originating from Amlodipine and Ramipril DKP from Ramipril) Use of placebo imparting pH of 6.2 was chosen finally since the level of Ramipril DKP was least in comparision the formulations having pH of Placebo of 6.4 & 6.7

There was significant contribution of pH of microenvironment around actives which was responsible for the stabilization of the combination product.

After stabilization of the product, the following experiments show how to achieve homogeneity and uniformity and development of a robust and scalable formulation.

### Example 4 :

| **S. No.** | **Ingredients** | **Mg/tablet** |
|---|---|---|
| 1 | Ramipril | 2.5 |
| 2 | Amlodipine Besylate | 7.00 |
| 3 | Hydroxy Propyl methyl cellulose | 0.44 |
| 4 | Pregelatinised Starch | 44.0 |
| 5 | Microcrystalline Cellulose | 45.56 |
| 6 | Sodium Stearyl fumarate | 0.50 |

### PROCEDURE:

1. Ramipril added as hydroxy propyl methyl cellulose granules.
2. Co-sift Ramipril granules with microcrystalline cellulose through #30 mesh sieve.
3. Co-sift Amlodipine Besilate & Pregelatinised starch & mix them with step 2 materials & blend for 5 min in blender to achieve homogeneity.
4. Lubricate the blend using sodium stearyl fumarate.
5. Compress the blend into tablets using suitable punches on a tablet press.
6. Tablets were packed in opaque white duplex and subjected for 6 month accelerated stablity studies which were analyzed at initial and 6 month intervals for percentage of potential degradants

In examples 4 and 5 the amounts of API and excipients are different from ex 1 to 3.

**Table No.4:**

| | **Uniformity of content in Ramipril+Amlodipine tablets 2.5/5 mg** | |
|---|---|---|
| | Amlodipine content | Ramipril content |
| Mean | 94.2 % | 108.6 % |
| % RSD | 5.0% | 3.5 % |

In-order to achieve proper homogeneity and improve the distribution of actives different mixing methodologies based on bulk densities were used.

The purpose here was to match the bulk densities of the Ramipril granules and extra granular portion of the formulation in-order to achieve proper homogeneity of active (especially Amlodipine).

Since the bulk density of Amlodipine and Microcrystalline cellulose was found to be 0.313 g/ml and 0.347 g/ml respectively, the purpose was to match with Ramipril granules whose density was found to be 0.277 g/ml.

Therefore increasing the bulk density of Ramipril granules was done by mixing with much bulkier excipient (herewith as pregelatinised starch) so that the bulk density of Ramipril granules and pregelatinised starch reaches near the bulk density of extra granular portion (herewith Amlodipine besilate+ microcrystalline cellulose).

The following trials with this methodology show how to achieve good homogeneity in the formulation.

### Examples 5:

| **S. No.** | **Ingredients** | **Mg/tablet** |
|---|---|---|
| 1 | Ramipril | 2.50 |
| 2 | Amlodipine Besylate | 7.00 |
| 3 | Hydroxy Propyl methyl cellulose | 0.44 |
| 4 | Pregelatinised Starch | 44.0 |
| 5 | Microcrystalline Cellulose | 45.56 |
| 6 | Sodium Stearyl fumarate | 0.50 |

### PROCEDURE:

1. Ramipril added as Hydroxy propyl methyl cellulose granules
2. Co-sift Ramipril granules with half portion of pregelatinised starch.
3. Co-sift Amlodipine Besilate with half portion of microcrystalline cellulose & mix them with step 2 materials & blend for 20 min in blender to achieve homogeneity.
4. Co-sift remaining portions of microcrystalline cellulose & pregelatinised starch through # 40 mesh.
5. Blended step 2,3 and 4 for 15 minutes at 18 RPM in a suitable blender.
6. Lubricated the blend of step 5 using sodium stearyl fumarate.
7. Compress the blend into tablets using suitable punches on a tablet press.

**Table No.5:**

| | **Uniformity of content in Ramipril+Amlodipine tablets 2.5/5 mg** | |
|---|---|---|
| | Amlodipine content | Ramipril content |
| Mean | 100.3% | 99.1% |
| % RSD | 1.2% | 1.6% |

### Example 6: Bioequivalence Dissolution profiles.

The dissolution profiles of the products of the invention are compared to the dissolution profiles of the references products. These studies are carried out per the multimedia dissolution profile study conditions established as followed.

Multi Media dissolution profile of Ramipril+Amlodipine tablets 10/5 mg

**Table no. 6**

| Amlodipine (%) drug release | | | | | |
|---|---|---|---|---|---|
| Time points (Mins) | 5 | 10 | 15 | 30 | 45 |
| 0.1N HCl 75 rpm 900 ml Paddle | | | | | |
| Istin 5mg | 95 | 97 | 97 | 96 | 97 |
| Istin 5mg | 97 | 97 | 97 | 96 | 96 |
| Example 1-Test product - Monolayer- | 87 | 86 | 86 | 88 | 87 |
| pH 4.5 Acetate buffer 75 rpm 900 ml Paddle | | | | | |
| Istin 5mg | 90 | 94 | 94 | 94 | 93 |
| Istin 5mg | 94 | 94 | 94 | 93 | 95 |
| Example 1-Test product - Monolayer | 91 | 93 | 92 | 91 | 92 |
| pH 6.8 Phosphate buffer 75 rpm 900 ml Paddle | | | | | |
| Istin 5mg | 79 | 87 | 87 | 88 | 90 |
| Istin 5mg | 80 | 88 | 89 | 90 | 91 |
| Example 1-Test product - Monolayer | 79 | 87 | 89 | 87 | 89 |
| Ramipril (%) drug release | | | | | |
| Time points (Mins) | 5 | 10 | 15 | 30 | 45 |
| 0.1N HCl 75 rpm 900 ml Paddle | | | | | |
| Cardace 10mg | 95 | 95 | 96 | 96 | 96 |
| Example 1-Test product - Monolayer | 104 | 104 | 104 | 106 | 106 |
| pH 4.5 acetate buffer 75 rpm 900 ml Paddle | | | | | |
| Cardace 10mg | 95 | 96 | 96 | 96 | 96 |
| Example 1-Test product - Monolayer | 99 | 100 | 100 | 100 | 101 |
| pH 6.8 Phosphate buffer 75 rpm 900 ml Paddle | | | | | |
| Cardace 10mg | 96 | 96 | 97 | 97 | 97 |
| Example 1-Test product - Monolayer | 98 | 99 | 99 | 100 | 100 |

## Claims

1. A stable solid oral pharmaceutical fixed dose composition in form of a tablet comprising Ramipril, amlodipine besilate and pharmaceutically acceptable excipients, wherein ramipril under the form of coated granules is embedded in an extragranular matrix comprising amlodipine besilate, wherein the pharmaceutically acceptable excipients are selected from the group consisting of diluent, disintegrant, antiadherent, binder lubricant and mixture thereof and wherein the pH of the excipient mixture, after dispersion in 50 ml of purified water, is from 6,1 to 6,3 preferably 6,2.

2. The composition according to claim 1 wherein the tablet is further package in duplex blister pack.

3. The composition according to any one of claims 1 to 2 wherein Ramipril represents between 2,0% and 20% by weight of the total composition, preferably 2,5% or 10% by weight of the total composition.

4. The composition according to any one of claims 1 to 3 wherein the amlodipine besilate represents between 5% and 10% by weight of the total composition and preferably 7.0%.

5. The solid composition according to any one of claims 1 to 4 wherein the amount of ramipril is comprised between 2,5mg and 10mg of the total weight of the tablet, preferably 2,5mg or 10mg.

6. The solid composition according to any one of claims 1 to 5 wherein the amount of amlodipine besilate is comprised between 5mg and 10mg of the total weight of the tablet, preferably 7mg.

7. The solid composition according to any one of claims 1 to 6 under the form of a tablet wherein the total weight of the tablet is between 80mg and 100mg, preferably 100mg.

8. The composition according to any one of claims 1 to 7 having less than 3.19% (w/w) of Ramipril DKP and Amlodipine Impurity-D of less than 0.14% (w/w) after 6 months at 40°C & 75% RH.

9. A process for the preparation of a stable oral pharmaceutical composition comprising Ramipril and amlodipine besilate, wherein the process comprises the steps of:
1) granulating Ramipril and one or more pharmaceutically acceptable excipients, with aqueous solution containing a binder, to form granules;
2) drying the granules;
3) separately blending amlodipine besilate with pharmaceutically acceptable excipients;
4) mixing the Ramipril granules of step ii with the amlodipine besilate blend of step iii);
5) lubricating the blend of step iv); optionally after a pre-lubricating step; and
6) compressing the mixture into tablets
wherein the group of pharmaceutically acceptable excipients used in steps 1) and 3) of the process are selected from the group consisting of diluent, disintegrant, antiadherent, binder lubricant and mixture thereof and wherein the pH of the excipient mixture, after dispersion in 50 ml of purified water, is from 6,1 to 6,3 preferably 6,2.

10. The process of claim 9 wherein the pharmaceutically acceptable excipients are selected from hydroxyl propyl methyl cellulose, pregelatinised starch, microcrystalline cellulose and sodium stearyl fumarate.

11. The process according to any one of claims 9 to 10 further comprising the step of coating the tablet and packaging in suitable duplex blister pack.

12. The process according to any one of claims 9 to 11 comprising the step of
1) add Ramipril as Hydroxy propyl methyl cellulose granules;
1a) Co-sift Ramipril granules with half portion of pregelatinised starch;
2) Co-sift Amlodipine Besilate with half portion of microcrystalline cellulose;
3) Mix step 3 material with step 2 materials & blend for 20 min in blender to achieve homogeneity;
3a) Co-sift remaining portions of microcrystalline cellulose & pregelatinised starch through # 40 mesh;
3b) Blend step 4 and 5 for 15 minutes at 18 RPM in a suitable blender;
4) Lubricate the blend of step 6 using sodium stearyl fumarate;
5) Compress the blend into tablets using suitable punches on a tablet press.

13. Use of Ramipril and Amlodipine besilate in the manufacture of a medicament for the treatment of arterial hypertension and prevention of other cardiovascular diseases such as myocardial infarction, cerebrovascular disorders and cardiac and renal insufficiency wherein said medicament is in the stable solid fixed dose composition according to anyone of claim 1 to 8.

## Patentansprüche

1. Stabile feste orale pharmazeutische Fixdosis-Zusammensetzung in Form einer Tablette, die Ramipril, Amlodipinbesilat und pharmazeutisch annehmbare Hilfsstoffe umfasst, worin Ramipril in Form eines beschichteten Granulats in einer Amlodipinbesilat umfassenden extragranularen Matrix eingebettet ist, worin die pharmazeutisch annehmbaren Hilfsstoffe aus der aus Verdünnungsmittel, Sprengmittel, Antihaftmittel, Bindemittel, Gleitmittel und Gemische davon bestehenden Gruppe ausgewählt sind und worin der pH-Wert des Hilfsstoffgemisches nach Dispersion in 50 ml gereinigtem Wasser 6,1 bis 6,3, vorzugsweise 6,2 beträgt.

2. Zusammensetzung nach Anspruch 1, worin die Tablette ferner in einer Duplexblisterverpackung verpackt ist.

3. Zusammensetzung nach einem der Ansprüche 1 bis 2, worin Ramipril zwischen 2,0 Gew.-% und 20 Gew.-% der Gesamtzusammensetzung, vorzugsweise 2,5 Gew.-% oder 10 Gew.-% der Gesamtzusammensetzung ausmacht.

4. Zusammensetzung nach einem der Ansprüche 1 bis 3, worin Amlodipinbesilat zwischen 5 Gew.-% und 10 Gew.-% der Gesamtzusammensetzung und vorzugsweise 7,0% ausmacht.

5. Feste Zusammensetzung nach einem der Ansprüche 1 bis 4, worin die Menge von Ramipril zwischen 2,5 mg und 10 mg des Gesamtgewichts der Tablette, vorzugsweise 2,5 mg oder 10 mg beträgt.

6. Feste Zusammensetzung nach einem der Ansprüche 1 bis 5, worin die Menge von Amlodipinbesilat zwischen 5 mg und 10 mg des Gesamtgewichts der Tablette, vorzugsweise 7 mg beträgt.

7. Feste Zusammensetzung nach einem der Ansprüche 1 bis 6 in Form einer Tablette, worin das Gesamtgewicht der Tablette zwischen 80 mg und 100 mg, vorzugsweise 100 mg beträgt.

8. Zusammensetzung nach einem der Ansprüche 1 bis 7, mit weniger als 3,19 Gew.-% Ramipril DKP und weniger als 0,14 Gew.-% Amlodipin Unreinheit-D nach 6 Monaten bei 40°C & 75% relativer Feuchte.

9. Verfahren zur Herstellung einer stabilen oralen pharmazeutischen Zusammensetzung, die Ramipril und Amlodipinbesilat umfasst, worin das Verfahren die folgenden Schritte umfasst:
1) Granulierung von Ramipril und einem oder mehreren pharmazeutisch annehmbaren Hilfsstoffen mit einer ein Bindemittel enthaltenden wässrigen Lösung zur Bildung von Granulat;
2) Trocknen des Granulats;
3) separates Vermischen von Amlodipinbesilat mit pharmazeutisch annehmbaren Hilfsstoffen;
4) Vermischen des Ramipril-Granulats aus Schritt 2) mit dem Amlodipinbesilat-Gemisch aus Schritt 3);
5) Fetten des Gemisches aus Schritt 4); fakultativ nach einem Vorfettungsschritt; und
6) Kompression des Gemisches zur Bildung von Tabletten, worin die Gruppe der in Schritt 1) und 3) des Verfahrens verwendeten pharmazeutisch annehmbaren Hilfsstoffe aus der aus Verdünnungsmittel, Sprengmittel, Antihaftmittel, Bindemittel, Gleitmittel und Gemische davon bestehenden Gruppe ausgewählt ist und worin der pH-Wert des Hilfsstoffgemisches nach Dispersion in 50 ml gereinigtem Wasser 6,1 bis 6,3, vorzugsweise 6,2 beträgt.

10. Verfahren nach Anspruch 9, worin die pharmazeutisch annehmbaren Hilfsstoffe unter Hydroxylpropylmethylcellulose, vorgelatinisierter Stärke, mikrokristalliner Cellulose und Natriumstearylfumarat ausgewählt sind.

11. Verfahren nach einem der Ansprüche 9 bis 10, ferner umfassend den Schritt der Beschichtung der Tablette und der Verpackung ein einer geeigneten Duplexblisterverpackung.

12. Verfahren nach einem der Ansprüche 9 bis 11, umfassend die folgenden Schritte:
1) Hinzufügen von Ramipril als Hydroxypropylmethylcellulose-Granulat;
1a) Sieben des Ramipril-Granulats mit der halben Portion vorgelatinisierter Stärke;
2) Sieben von Amlodipinbesilat mit der halben Portion mikrokristalliner Cellulose;
3) Vermischen des Materials aus Schritt 3 mit dem Material aus Schritt 2 und homogenes Vermischen in einem Mischer für 20 Minuten;
3a) Sieben der restlichen Portionen von mikrokristalliner Cellulose und vorgelatinisierter Stärke durch ein Sieb # 40;
3b) Vermischen von Schritt 4 und 5 in einem geeigneten Mischer für 15 Minuten bei 18 U/min;
4) Fetten des Gemisches aus Schritt 6 mit Natriumstearylfumarat;
5) Kompression des Gemisches zur Bildung von Tabletten unter Verwendung von geeigneten Stempeln auf einer Tablettenpresse.

13. Verwendung von Ramipril und Amlodipinbesilat bei der Herstellung eines Medikaments zur Behandlung von arterieller Hypertonie und zur Verhinderung von anderen kardiovaskulären Erkrankungen, wie beispielsweise Myokardinfarkt, zerebrovaskuläre Störungen und Herz- und Niereninsuffizienz, worin das Medikament eine stabile feste Fixdosis-Zusammensetzung nach einem der Ansprüche 1 bis 8 ist.

## Revendications

1. Composition pharmaceutique orale à dose fixe, solide et stable, sous la forme d'un comprimé, comprenant du ramipril, du bésylate d'amlodipine et des excipients pharmaceutiquement acceptables, le ramipril sous la forme de granules enrobés étant incorporé dans une matrice extragranulaire comprenant du bésylate d'amlodipine, les excipients pharmaceutiquement acceptables étant choisis dans le groupe constitué d'un diluant, un délitant, un antiadhérent, un liant, un lubrifiant et un mélange de ceux-ci et le pH du mélange d'excipients, après dispersion dans 50 ml d'eau purifiée, étant de 6,1 à 6,3, de préférence de 6,2.

2. Composition selon la revendication 1, dans laquelle le comprimé est en outre emballé dans une plaquette blister double coque.

3. Composition selon l'une quelconque des revendications 1 et 2, dans laquelle le ramipril représente entre 2,0 % et 20 % en poids de la composition totale, de préférence 2,5 % ou 10 % en poids de la composition totale.

4. Composition selon l'une quelconque des revendications 1 à 3, dans laquelle le bésylate d'amlodipine représente entre 5 % et 10 % en poids de la composition totale et de préférence 7,0 %.

5. Composition solide selon l'une quelconque des revendications 1 à 4, dans laquelle la quantité de ramipril est comprise entre 2,5 mg et 10 mg du poids total du comprimé, de préférence 2,5 mg ou 10 mg.

6. Composition solide selon l'une quelconque des revendications 1 à 5, dans laquelle la quantité de bésylate d'amlodipine est comprise entre 5 mg et 10 mg du poids total du comprimé, de préférence 7 mg.

7. Composition solide selon l'une quelconque des revendications 1 à 6 sous la forme d'un comprimé, le poids total du comprimé étant compris entre 80 mg et 100 mg, de préférence 100 mg.

8. Composition selon l'une quelconque des revendications 1 à 7 ayant moins de 3,19 % (m/m) de ramipril-DKP et une impureté D d'amlodipine de moins de 0,14 % (m/m) après 6 mois à 40 °C et 75 % HR.

9. Procédé pour la préparation d'une composition pharmaceutique orale stable comprenant du ramipril et du bésylate d'amlodipine, le procédé comprenant les étapes consistant à
1) granuler du ramipril et un ou plusieurs excipients pharmaceutiquement acceptables, avec une solution aqueuse contenant un liant, pour former des granules ;
2) sécher les granules ;
3) mélanger séparément le bésylate d'amlodipine avec des excipients pharmaceutiquement acceptables ;
4) mélanger les granules de ramipril de l'étape ii) avec le mélange de bésylate d'amlodipine de l'étape iii) ;
5) lubrifier le mélange de l'étape iv) ; facultativement après une étape de prélubrification ; et
6) presser le mélange en comprimés,
le groupe d'excipients pharmaceutiquement acceptables utilisés dans les étapes 1) et 3) du procédé étant choisis dans le groupe constitué d'un diluant, un délitant, un antiadhérent, un liant, un lubrifiant et un mélange de ceux-ci et le pH du mélange d'excipients, après dispersion dans 50 ml d'eau purifiée, étant de 6,1 à 6,3, de préférence de 6,2.

10. Procédé de la revendication 9, dans lequel les excipients pharmaceutiquement acceptables sont choisis parmi l'hydroxypropylméthylcellulose, l'amidon prégélatinisé, la cellulose microcristalline et le stéarylfumarate de sodium.

11. Procédé selon l'une quelconque des revendications 9 et 10, comprenant en outre l'étape d'enrobage du comprimé et l'emballage dans une plaquette blister double coque adaptée.

12. Procédé selon l'une quelconque des revendications 9 à 11, comprenant les étapes consistant à
1) ajouter du ramipril sous forme de granules d'hydroxypropylméthylcellulose,
1a) co-tamiser des granules de ramipril avec une demi-portion d'amidon prégélatinisé,
2) co-tamiser du bésylate d'amlodipine avec une demi-portion de cellulose microcristalline,
3) mélanger le matériau de l'étape 3 avec les matériaux de l'étape 2 et mélanger pendant 20 min dans un mélangeur pour obtenir l'homogénéité,
3a) co-tamiser les parties résiduelles de cellulose microcristalline et d'amidon prégélatinisé à travers un tamis n° 40,
3b) mélanger les étapes 4 et 5 pendant 15 minutes à 18 tours/min dans un mélangeur adapté,
4) lubrifier le mélange de l'étape 6 en utilisant du stéarylfumarate de sodium,
5) presser le mélange en comprimés en utilisant des poinçons adaptés sur une presse à comprimés.

13. Utilisation du ramipril et du bésylate d'amlodipine dans la fabrication d'un médicament pour le traitement de l'hypertension artérielle et la prévention d'autres maladies cardiovasculaires telles que l'infarctus du myocarde, les troubles cérébrovasculaires et l'insuffisance cardiaque et rénale, ledit médicament étant dans la composition à dose fixe solide stable selon l'une quelconque des revendications 1 à 8.
